# EUROPEAN PATENT SPECIFICATION

(11) **EP 3 818 948 B1**
(45) Date of publication and mention of the grant of the patent: **10.04.2024**
(21) Application number: 20206899.5
(22) Date of filing: 11.11.2020
(51) Int. Cl.: A61B 17/04, A61B 17/06

(54) **REPAIR DEVICE FOR DEPLOYING ANCHORS INTO TISSUE**
REPARATURVORRICHTUNG ZUM EINSETZEN VON ANKERN IN GEWEBE
DISPOSITIF DE RÉPARATION POUR LE DÉPLOIEMENT D'ANCRAGES DANS DES TISSUS

(30) Priority: 11.11.2019 US 201962933777 P; 29.07.2020 US 202063058132 P
(43) Date of publication of application: 12.05.2021
(73) Proprietor: Zimmer, Inc., Warsaw, IN 46580 (US)
(72) Inventor: LAWHORN, Keith Willis, Fairfax, VA 22033 (US); NORTON, Daniel R., Warsaw, IN 46580 (US)
(74) Representative: Venner Shipley LLP

(56) References cited:
- WO-A1-2019/102484
- US-A1- 2008 140 093
- US-A1- 2016 015 376
- US-A1- 2017 290 579

## Description

### FIELD OF THE DISCLOSURE

The present disclosure relates to tissue repair devices for bone or tissue repair surgery such as a meniscal repair surgery.

### BACKGROUND OF THE DISCLOSURE

In the human body, bone or tissue can require repair. A meniscus is a fibrocartilaginous structure found within a joint, such as a knee joint. Forceful twisting or rotation of the knee (or other joint) can tear or otherwise damage the meniscus. A surgical repair of the meniscus may be required.

US 2016/015376 A1 discloses a suturing apparatus for joining of biological tissues.

### SUMMARY

The invention is defined in independent claim 1. Certain optional features of the invention are defined in the dependent claims. The invention is shown in figure 13. The embodiments of figures 5,6,8 and 14 do not form part of the invention.

### BRIEF DESCRIPTION OF THE DRAWINGS

FIG. 1 illustrates a portion of a tissue repair device accessing a knee joint to perform a meniscal repair via a lumen in accordance with one example of the present application.
FIGS. 2A and 2B are highly schematic views of a tissue repair device, particularly an anchor of the device being directed to a tibia with tears in a meniscus thereof, according to an example of the present application.
FIG. 3 is a perspective view of the tibia with two anchors connected by a suture loop being used to repair a tear in the meniscus thereof, according to an example of the present application
FIG. 4 is a perspective view of the tissue repair device prior to use, according to one example of the present application.
FIGS. 5A-5D are highly schematic enlarged views of a distal end portion of the tissue repair device such as that of FIG. 4, in four sequential stages of use, according to one example of the present application.
FIG. 6 is a highly schematic enlarged view of the distal end portion of the tissue repair device such as that of FIG. 4, according to another example of the present application.
FIG. 7A is a side view a second tissue repair device prior to use, according to one example of the present application.
FIG. 7B is a side view of a third tissue repair device prior to use, according to another example of the present application.
FIGS. 8A-8D are highly schematic enlarged views of the distal end portion of the tissue repair device such as that of FIG. 4 or another example disclosed herein, in four sequential stages of use, according to one example of the present application.
FIGS. 9A and 9B are plan view and an enlarged view from of a fourth tissue repair device prior to use, according to one example of the present application.
FIGS. 10A and 10B are plan views from different sides of a fifth tissue repair device prior to use, according to one example of the present application.
FIGS. 11A-11C are plan views and a cross-sectional view of a sixth tissue repair device prior to use, according to one example of the present application.
FIG. 12 is a highly schematic enlarged view of the distal end portion of the tissue repair devices disclosed herein, according to another example of the present application.
FIGS. 13A-13C are highly schematic enlarged views of the distal end portion of any of the tissue repair devices disclosed herein, in three sequential stages of use, according to one example of the present application.
FIGS. 14A-14D are highly schematic enlarged views of the distal end portion of any of the tissue repair devices disclosed herein, in four sequential stages of use, according to one example of the present application.

Corresponding reference characters indicate corresponding parts throughout the several views. Inserters in the drawings are not necessarily drawn to scale. The configurations shown in the drawings are merely examples, and should not be construed as limiting the scope of the invention in any manner.

### DETAILED DESCRIPTION

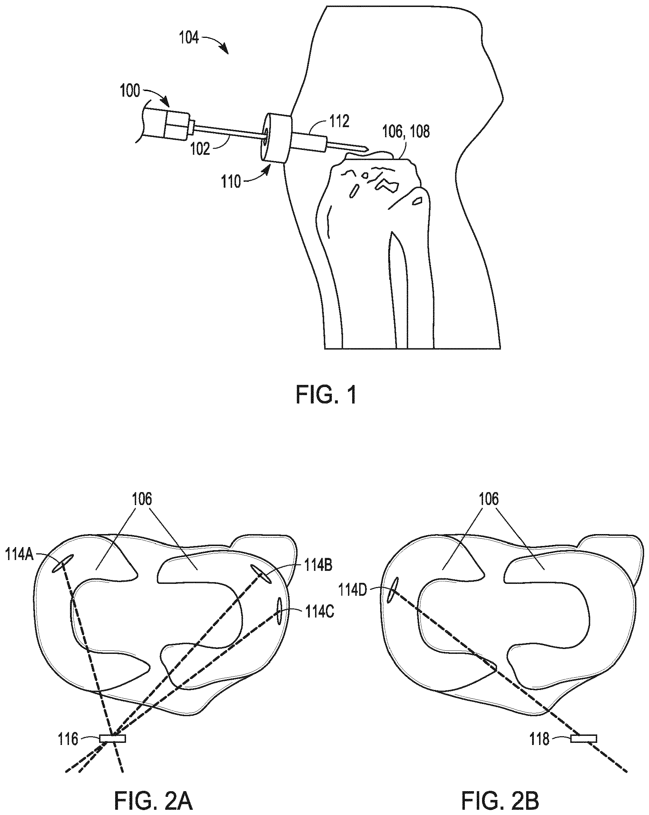

To repair tissue (including both soft tissue and bone) in the human body a surgeon can deploy two soft anchors connected by a loop of suture. For example, the two soft anchors and the loop of suture can be utilized to repair a tear in a meniscus. These anchors are referred to as "soft" herein as they are formed of material(s) that are flexible and/or deformable, such as a suture sleeve or other suture material. The surgeon can use a repair device with a needle with a passageway to pierce the tissue on one side of the tear, deploy a first of the two anchors, pull the repair device back through the tissue, pierce the tissue with the repair device on the other side of the tear, and deploy a second of the two anchors. The surgeon can then pull on the suture to draw the two deployed anchors together, which can close the tear. The surgeon can then cut the suture. This process is further illustrated and described reference to FIGS. 1-3.

There is ongoing effort to improve tissue repair devices, such as making them easier to use and have them provide for more repeatable results during a repair procedure. The meniscal repair device discussed below in further detail can provide improvements in these and other areas. For example, the present meniscal repair device uses an anchor formed from a deformable material. This can be preferable to using a hard-plastic anchor, made from a relatively hard material, such as polyether ether ketone (PEEK). For example, in cases where the suture pulls through the meniscal tissue, the anchor can be dislodged within the knee joint space. A hard-plastic anchor can cause joint damage by moving within the joint space. Chances of damage can be reduced with a soft anchor. Furthermore, the present meniscal repair device uses two suture strands across the tissue, rather than a single suture strand, which can distribute the suture force over a larger tissue area and therefore reduce the likelihood that suture will rip through the tissue. The tissue repair devices of the present can deploy the anchors without using any pre-tied suture knots, which could rub against the femur.

Tissue repair devices for deploying soft anchors connected with the suture loop, such as those of Application Serial No. 15/482,106 (now U.S. Patent No. 10,499,902) are known.

The present application provides further examples of tissue repair devices that can be utilized for deploying such soft anchors. One or more of the tissue repair devices disclosed herein may be preferred by a physician according to the physician's individual requirements or preferences.

FIGS. 1-3 show views of various stages of an example of how a surgeon can use a tissue repair device, such as a repair device 100 (FIG. 1) for a meniscal repair. This is but one example of a surgical repair procedure that can utilize the devices disclosed herein; other suitable procedures can utilize the disclosed tissue repair devices. Further details of a procedure to repair the meniscus are discussed in Application Serial No. 15/482,106.

As illustrated in FIG. 1, a surgeon can position the repair device 100 such that a needle 102 thereof can enter a knee joint 104 of a patient. More particularly, the needle 102 can be advanced to be positioned just above a meniscus 106. The needle 102 can be advanced parallel to or above a tibial joint surface 108, for example. According to the example of FIG. 1, passage of the needle 102 through at least some of the tissue of the knee joint 104 to the can be assisted by an inserter lumen 110. The inserter lumen 110 can be specifically sized and otherwise configured for assisting passage of the needle 102 through the tissue of the knee joint 104. As such, the inserter lumen 110 can have a passageway 112 with a longitudinal length according to some examples. According to some examples, the inserter lumen 110 can be available as part of a system with the system having a plurality of inserter lumens of different longitudinal lengths for different knee joint anatomy. The inserter lumen 110 can additionally assist in reducing leakage of bodily fluid from the knee joint 104. The inserter lumen 110 can be substantially straight along a longitudinal passage 112 thereof. However, the inserter lumen 110 along a distal end of the longitudinal passage 112 can be angled/curved such as by up to about 25 degrees relative to a remainder of the longitudinal passage 112. This curvature/angulation can facilitate a desired curvature of the needle 102. According to further examples, the needle 102 can be curved at a distal end thereof as will be discussed subsequently.

The surgeon can avoid placing the access portal and inserter lumen 110 too superior or inferior of the tibial joint surface 108 and can ensure that the portal is large enough to readily pass instruments including a suture cutter therethrough. The surgeon can measure a distance from a back side of the meniscus to a desired needle penetration point at the repair site using a meniscal depth gauge. The surgeon can utilize a probe through the access portal to help determine whether a straight or curved needle will position the soft anchors in a more optimal manner. Selecting between a straight or curved needle can depend on the location of the tear, and on the location of the portal (e.g., the location of the skin incision). It can be preferable to choose the needle 102 with a shape so that the needle 102 emerges through the back of the tissue and not the underside of the tissue.

FIG. 2A shows an approach to access posterior horn tears 114A, 114B and 114C of the meniscus 106 from a medial portal 116. The tears 114A, 114B and 114C are illustrated exemplary as including both medial and lateral tears.

FIG. 2B shows that the surgeon can approach a mid-body tear 114D of the meniscus 106 from a contralateral portal 118. To adjust a needle depth, if needed, the surgeon can push down on a depth control slider in a forward motion to decrease the needle length exposed.

Once tears are identified and an access portal created, the surgeon can position the repair device 100 (FIG. 1) for executing a repair as further discussed in Application Serial No. 15/482,106. This process can include advancing the needle until a tip thereof penetrates the meniscus. Optionally, the surgeon can rotate the repair device (about a longitudinal axis of the meniscal repair device) . The surgeon can then advance the needle just beyond the meniscocapsular junction. Next, the surgeon can deploy a first soft anchor from the repair device (e.g., push the first soft anchor out of the repair device). This deployment process will be discussed and illustrated in further detail subsequently. The repair device 100 can be configured to advance a second soft anchor forward into a position where it is able to be deployed. This position for the second soft anchor can be a position previously occupied by the first soft anchor. The surgeon can pull the needle tip gently out of the meniscus.

The surgeon can then begin the process of deploying the second soft anchor. The surgeon can reposition the needle tip at a desired location and can advance the needle tip as described above to beyond the meniscocapsular junction. The surgeon can then deploy the second soft anchor (e.g., push the second soft anchor out of the repair device). This deployment process will be discussed and illustrated in further detail subsequently. The surgeon can then remove the repair device from the knee joint.

The surgeon can choose to position the two anchors in a suitable pattern that is matched to the particular tear of the meniscus. In some examples, the surgeon can position one anchor above the other, in a so-called horizontal mattress pattern. In other examples, the surgeon can position the anchors side by side, in a so-called vertical mattress pattern. The vertical mattress stitching pattern can be well-suited for meniscal repairs due to its ability to achieve deep and superficial wound closure, edge eversion and precise vertical alignment of the superficial wound margins. The surgeon can insert the first anchor on the inferior meniscal rim. The surgeon can insert the second anchor superior to the tear on the meniscal rim. Implants in this superior meniscal location can require shorter distances of deployment, since the depth of meniscus can be less than the depth at the inferior location. To decrease the needle depth for the superior position, the surgeon can adjust an adjustable depth stop until the needle reaches a desired depth .

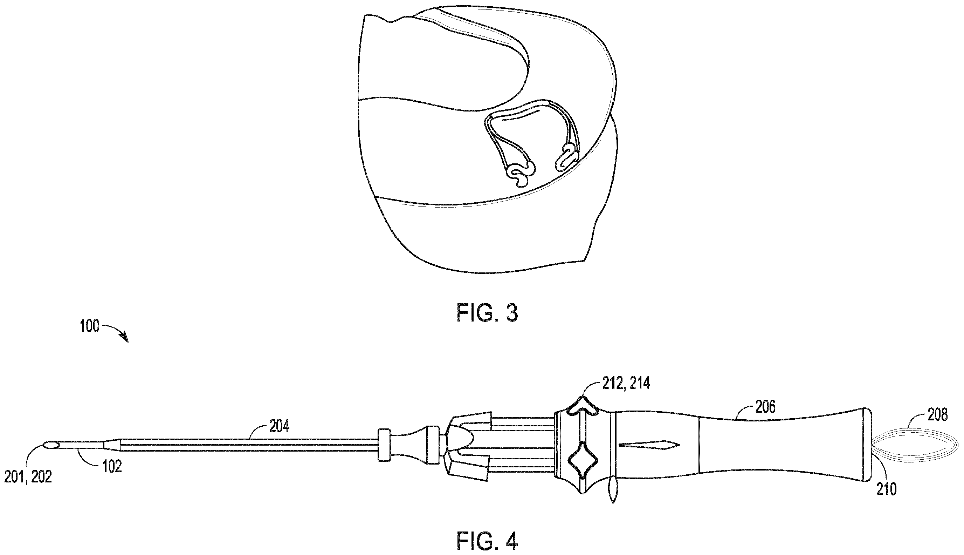

After deployment of the second soft anchor, the surgeon can tension sutures that connect the first and second anchors together. For example, after the surgeon retracts the repair device from the joint, a suture loop and a single strand of suture can protrude from the access portal. The suture loop and single strand of suture can be formed from a single piece of suture run through itself and configured as an adjustable loop. If the surgeon pulls on the loop, the loop does not increase in size. If the surgeon pulls on the single strand of suture, the loop shrinks. Deploying the anchors as described above can produce a small loop of suture inside the joint, a larger loop of suture emerging from the skin, and a free strand of suture emerging from the skin. The surgeon can pull on the larger loop to set the anchors at the repair site as desired and can pull the single strand to contract the large loop down to the surface of the meniscus such as illustrated in FIG. 3. The surgeon can visually confirm, with a scope, that the anchor is fully set at the repair site such as in the manner illustrated in FIG. 3. The surgeon can pull on the strand until tension on the second loop matches the tension of the first loop. If desired, the surgeon can use a probe to check the repair site for appropriate tension. Finally, the surgeon can cut the suture. The surgeon can insert a cutter into the access portal and advance the cutter to sever the suture. The combination of the anchors and remaining suture can work together to close the tear as illustrated in FIG. 3.

FIG. 4 shows a perspective view of an example of the repair device 100, in accordance with some examples. The repair device 100 can include inserters previously shown in FIG. 1, such as the needle 102. However, the repair device 100 can have additional inserters from those of FIG. 1.

The repair device 100 includes the needle 102 at a distal end 200 thereof. The needle 102 can be shaped as a cylindrical tube, with a sharp tip 201 designed to pierce tissue. The sharp tip 201 can be angled to penetrate tissue. In some examples, the needle 102 at or adjacent the distal end 200 can be angled as previously discussed. The needle 102 can define a lumen 202 comprising a bore that can extend therethrough along a longitudinal length of the needle 102 to adjacent the tip 201. In some examples, the needle 102 and lumen 202 can be shaped so that when a surgeon advances the needle 102 through tissue, the needle 102 pierces the tissue without clogging the lumen 202. The repair device 100 can store one or more of the soft anchors, sutures and portions an anchor deployment mechanism in the lumen 202 of the needle 102 according to some examples of the present application. However, others of the soft anchors, sutures and the portions of the anchor deployment mechanism can also be housed in other portions of the repair device 100 than the needle 102.

According to some examples, the needle 102 can be selectively retractable such as back into an outer tube 204 or other structure (e.g., part of the handle, etc.) as will be further discussed and illustrated herein. In some examples, a distal end of the outer tube 204 can be configured as a stop or blunt surface for contacting tissue. The outer tube 204 can be provided with an internal stop to halt retraction of the needle 102. The repair device 100 can have actuator(s) for retracting and/or extending the needle 102. The longitudinal length of the needle 102 from the outer tube 204 can vary from between about 10 mm and about 100 mm. However, other suitable lengths for the needle 102 are contemplated and this length can change according to the type of surgery being performed.

The outer tube 204 can be coupled to and can receive a proximal portion of the needle 102. The needle 102 can extend internally within outer tube 204 in some cases. The outer tube 204 can be an optional component of the repair device 100 and may not be utilized in all examples.

The outer tube 204 and/or needle 102 can extend proximally to couple with a handle 206. The handle 206 can be configured to be gripped by a surgeon. In some cases, the handle 206 can be configured to receive sutures 208 such as those illustrated in FIG. 4. These sutures 208 can comprise the adjustable loop(s) and/or strand coupling the first soft anchor with the second soft anchor as previously described and illustrated. In the example of FIG. 4, the handle 206 can included a lumen or passageway allowing the sutures 208 to extend through the handle 206 to a proximal end 210 of the handle 206. The sutures 208 can extend from this proximal end 210 according to the example of FIG. 4.

A portion of the anchor deployment mechanism 212 comprising a push button 214 can be a part of the handle 206. The push button 214 can comprise an actuator for deploying the anchors as further discussed and illustrated herein. The push button 214 can be slidable proximally and distally with respect to the handle 206 between various positions. However, other types of actuators are contemplated. Other portions of the anchor deployment mechanism 212 such as a member (not illustrated) internal to the needle 102 can be configured to selectively couple with the soft anchors and can selectively move them for deployment as desired.

During use of the repair device 100, the surgeon pierces tissue with the needle 102, guides the needle 102 to a suitable location and orientation, then deploys the first anchor. To deploy the first anchor, the surgeon distally advances push button 214 with respect to a handle 206, for example. The needle 102 can then be removed from the tissue or repositioned. The process can be repeated to deploy the second anchor.

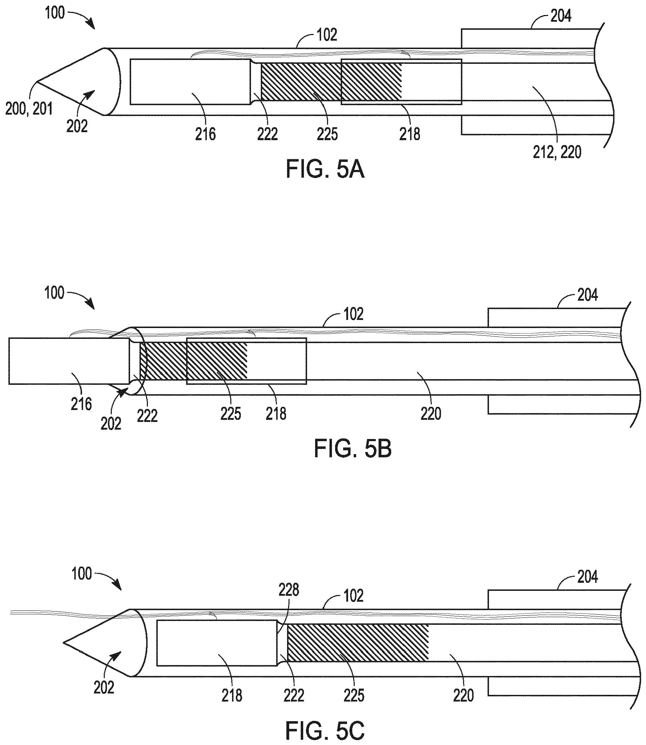

FIG. 5A is an enlarged highly schematic view of the distal end portion of the repair device 100 with a first anchor 216 and a second anchor 218 in the needle 102 prior to being deployed. As previously discussed, the needle 102 can define the lumen 202 extending through the needle 102 to adjacent the distal end 200 (adjacent the tip 201) of the needle 102. The needle 102 can couple with the outer tube 204 (or another element such as the handle) at a proximal portion. The first anchor 216 can be disposed in the lumen 202 of the needle 102 proximal to a distal end of the lumen 202. The second anchor 218 can be positioned in the lumen 202 proximal to the first anchor 216 and spaced therefrom. In some examples, the first 216 and second 218 anchors are placed into the lumen 202 during assembly of the repair device 100, so that the repair device 100 can be sold or purchased in an assembled state, prior to use. In other examples, the first 216 and second 218 anchors can be packaged separately and can be inserted into the repair device 100 prior to use.

As discussed previously, the anchors 216, 218 can be "soft" (i.e., they can be made from a relatively soft material such as fabric or suture), and thus they can bend, flex and/or deform under the force of a suture or other inserter(s) on the repair device 100. In some examples, the anchors 216, 218 can be nominally shaped as cylinders, or tubes having a circular or elongated cross-section, but can be configured to deform during assembly into the repair device 100 and/or during deployment. FIG. 3 shows an example of the repair devices being deformed after deployment.

Many of the FIGURES herein illustrate components of the repair device including the anchors in a highly schematic manner. This is done to better illustrate interaction of various components of the repair device. As an example, the anchors 216, 218 are illustrated as rectangles. However, it is recognized that the anchors 216, 218 can have other shapes and can be deformable as discussed above. Similarly, other components of the repair device can have shapes different from those illustrated herein.

According to other examples contemplated herein one or both of the anchors 216, 218 may not be soft and can be made from non-deformable material such as a hard plastic, etc. Thus, according to some examples the anchor(s) can have a cross-section or other geometry that is invariant. In these examples, the anchor(s) can have a hollow interior formed by a tubular shape of the anchor(s).

Although two anchors are illustrated, it is contemplated that further anchors could be used with and stored in the repair device 100. Additionally, the anchors 216, 218 can be differently sized/shaped relative to one another and/or can be formed of different material relative to one another according to some examples.

FIG. 5A shows a second portion of the anchor deployment mechanism 212 comprising an inserter 220. The inserter 220 can be configured to reside within the lumen 202 of the needle 102. According to one example, the inserter 220 can comprise a wire, thin shaft, tube or other mechanism capable of engaging and deploying the anchors 216, 218. The inserter 220 can be constructed of a flexible material that can generally conform with a shape (e.g., angle) of the lumen 202. Additionally or alternatively, the inserter 220 can be laser-cut to impart additional flexibility to the inserter 220, so that the 220 can more easily navigate a curved distal end of the needle 102. The inserter 220 can be movable relative to the needle 102 according to some examples such as those of FIG. 5A. Thus, the inserter 220 can be actuated from a proximal portion of anchor deployment mechanism 212. However, according to other examples, the inserter 220 can be stationary while other components such as the needle 102, etc. can be moveable relative thereto. According to some examples, the inserter 220 can have an outer diameter smaller than an inner diameter of at least the second anchor 218, so that the inserter 220 can pass therethrough as shown in FIG. 5A.

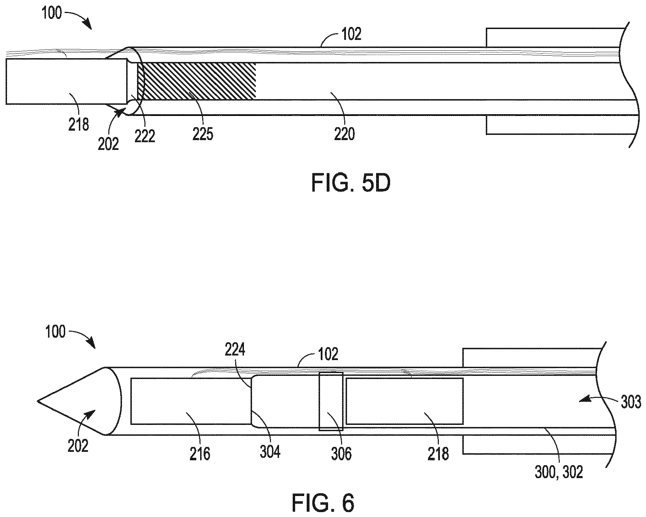

As shown in FIG. 5A, the inserter 220 can include a distal end 222 that can be blunt so as to be flared or can be otherwise configured to have a larger diameter than a remainder of the diameter of an elongated length of the inserter 220. Distal end 222 can be tapered such that the diameter increases moving distally. This shape can facilitate movement of the second anchor 218 over and distally past the distal end 222 as shown in FIG. 5D. According to further examples, the distal end 222 can have additional engagement features such as projections, teeth, etc. to facilitate engagement. The larger diameter of the distal end 222 can allow the inserter 220 to engage with a proximal end 224 of the first anchor 216. From this engaging position, the inserter 220 can act to push the first anchor 216 distally from the needle 102 as further illustrated in FIG. 5B.

FIGS. 5B-5D illustrate deployment of the first anchor 216 and then the second anchor 218 from the repair device 100. FIG. 5B shows the inserter 220 being advanced distally to move the first anchor 216 distally from the needle 102 via the opening to the lumen 202.

As shown in the example of FIG. 5B, the inserter 220 can include a thread 225 along a diameter thereof configured to engage with the second anchor 218. Although illustrated as the thread 225, according to further examples features such as teeth, projection(s) etc. are contemplated rather than or in addition to the thread 225. For example, a flared or larger diameter section of the inserter 220 such as occurs at the distal end 222 can be utilized proximally of and engaging a proximal end of the second anchor 218. This larger diameter section or feature can engage and advance the second anchor 218 into the position of FIGS. 5B and 5C. It is contemplated that in other examples the thread 225 or other feature can be part of the outer diameter of the lumen 202 in addition to or in alternative to being part of the inserter 220. As shown in FIG. 5B, the thread 225 can act to advance the second anchor 218 and/or retain the second anchor 218 in the advanced position of FIG. 5B and 5C. The advanced position of the second anchor 218 can be the same position within the lumen 202 as previously occupied by the first anchor 216.

According to the example of FIGS. 5B, the second anchor 218 can be provided with a mating thread (not shown) along an inner cavity thereof. The inserter 220 can be rotated in addition to being linearly advanced as shown in FIG. 5B. Such rotation, with the mating of threads can act to advance the second anchor 218 distally along the inserter 220 even as the inserter 220 is being advanced linearly as shown in FIG. 5B. When the inserter 220 is again retracted proximally as shown in FIG. 5C, the thread 225 can be configured to disengage, release or otherwise decouple from engagement with the thread (not shown) of the second anchor 218.

FIG. 5C shows the second anchor 218 within the lumen 202 proximate of the distal end 200 in a position such as that as previously occupied by the first anchor 216. The inserter 220 can be retracted proximate of the second anchor 218. Thus, the distal end 222 of the inserter 220 (with the larger diameter) can facilitate engagement of the inserter 220 with a proximal end 228 of the second anchor 218. From this engaging position, the inserter 220 can act to push the second anchor 218 distally from the needle 102 as further illustrated in FIG. 5D.

FIG. 6 shows an alternative example of an inserter 300 for the repair device 100 that can be used with the needle 102, the lumen 202, the first anchor 216 and the second anchor 218. In the example of FIG. 6, the inserter 300 comprises a tube 302 having an inner cavity 303 that has an inner diameter larger than the outer diameter of at least the second anchor 218. The inserter 300 can have features similar to those of the inserter 220 but along the inner cavity 303 thereof. Thus, the inserter 300 can include a distal end 304 that can be blunt so as to be flared or can be otherwise configured to have a smaller diameter than a diameter of a remainder of the elongated inner cavity 303 of the inserter 300.

The distal end 304 can be tapered such that the diameter decreases moving distally. This shape can facilitate movement of the second anchor 218 through and distally past the distal end 304 (to a position such as shown in FIG. 5D). According to further examples, the distal end 304 can have additional engagement features such as projections, teeth, etc. to facilitate engagement. The smaller diameter of the distal end 304 can allow the inserter 300 to engage with the proximal end 224 of the first anchor 216. From this engaging position, the inserter 300 can act to push the first anchor 216 distally from the needle 102 (such as was previously illustrated in FIG. 5B).

The example of FIG. 6 differs from that of FIGS. 5A-5D in that rather than a thread along the inserter 300, the repair device 100 can include a stop 306. This stop 306 can comprise a removable insert or other element made of plastic or other material. Upon deployment of the first anchor 216 with the inserter 300 extended, the stop 306 can be exposed sufficiently for removal. Removal of the stop 306 could also be facilitated by a dedicated instrument or features of the repair device 100. Upon removal of the stop 306, the second anchor 218 is then free to advance distally. This can occur during (and/or after) deployment of the first anchor 216. With the stop 306 removed, the inserter 300 can be configured to advance the second anchor 218 relative to the needle 102 and the inserter 300 to a position adjacent the distal end of the lumen 202 of the needle 102 such as that previously occupied by the first anchor 216. In some cases, with the stop 306 removed, the second anchor 218 is freely moveable and need not be advanced by the inserter 300 until moved distally past the distal end 304.

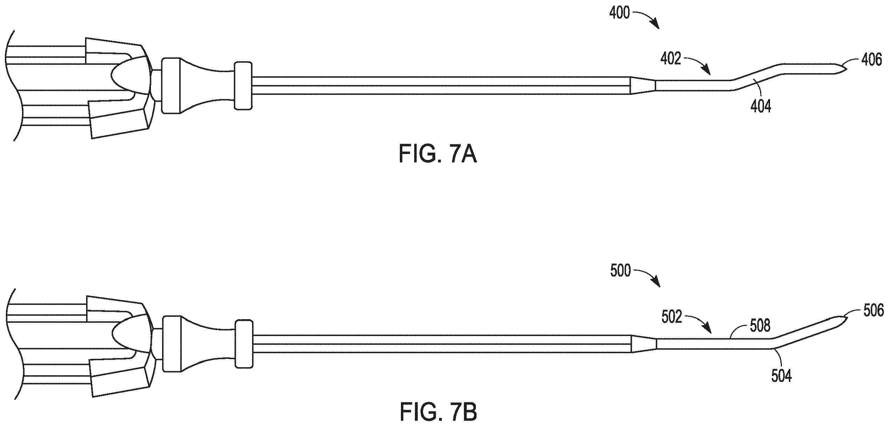

FIGS. 7A and 7B show examples of a second repair device 400 and a third repair device 500, respectively. The second repair device 400 of FIG. 7A includes a needle 402 having a curve at a middle section 404 proximal a distal end 406. This curve can displace the distal end 406 including the tip to be offset a distance relative to an elongate axis of a proximal section 408 of the needle 402. Such lateral distance can be on the order of about 1.5 mm to about 40 mm.

The third repair device 500 of FIG. 7B includes a needle 502 with a gentle curvature of a distal section 504. The curvature continues to a distal end 506. The curvature can be relative to an elongate axis of a middle section 508 of the needle 502. This curvature can be between 5 degrees and 25 degrees and can be about 10 mm to about 40 mm in longitudinal extent. Curvature in the manner of FIG. 7B can facilitate the needle 502 passing under a curvature of a condyle of a femur of the knee joint.

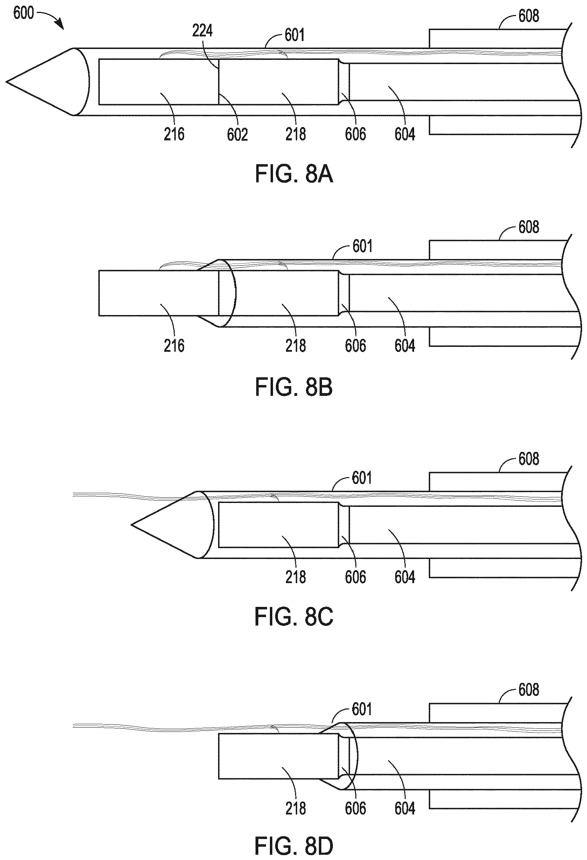

FIG. 8A shows another example of a distal portion of a repair device 600 with the first anchor 216 and second anchor 218 in the non-deployed positions within a needle 601 thereof. FIGS. 8B-8D illustrate deployment of the first anchor 216 and then the second anchor 218.

The repair device 600 differs from that of the repair device 100 of FIGS. 5A-6 in several respects. First, the first anchor 216 can abut the second anchor 218. More particularly, the proximal end 224 of the first anchor 216 can be in direct contact with a distal end 602 of the second anchor 218. Furthermore, an inserter 604 of the repair device 600 can be initially positioned proximal of the second anchor 218 rather than the first anchor 216 as was previously been the case with the device 100 of FIGS. 5A-6. A distal end 606 of the inserter 604 can be configured in the manner of those discussed previously so as to push the first anchor 216 and the second anchor 218 from the needle 601 of the repair device 600. However, the inserter 604 differs in that the inserter 604 can be stationary, and therefore, may not move relative to the needle 601. Thus, the inserter 604 can be coupled with the handle 608 and/or with another non-moving feature of the repair device 600.

Additionally, the example of FIGS. 8B-8D illustrate that the needle 601 can be configured to be selectively retractable and extendable relative to other elements such as the inserter 604, the handle 608 and/or other features (e.g., the outer tube of FIG. 5A, etc.). Thus, the needle 601 can be moveable according to the example of FIGS. 8A-8D. FIG. 8A shows the needle 601 in an extended position relative to the inserter 604, the handle 608, etc. FIG. 8B shows the needle 601 moved to the retracted position relative to the inserter 604 and the handle 608. In the retracted position, the needle 601 can be received in the handle 608, for example. Retraction of the needle 601 can deploy the first anchor 216 as shown in FIG. 8B.

FIG. 8C shows the needle 601 selectively moved back to the extended position with the needle 601 retaining the second anchor 218. FIG. 8D shows the needle 601 moved to the retracted position relative to the inserter 604 and the handle 608. In the retracted position, the needle 601 can be received in the handle 608, for example. Retraction of the needle 601 can deploy the second anchor 218 as shown in FIG. 8D.

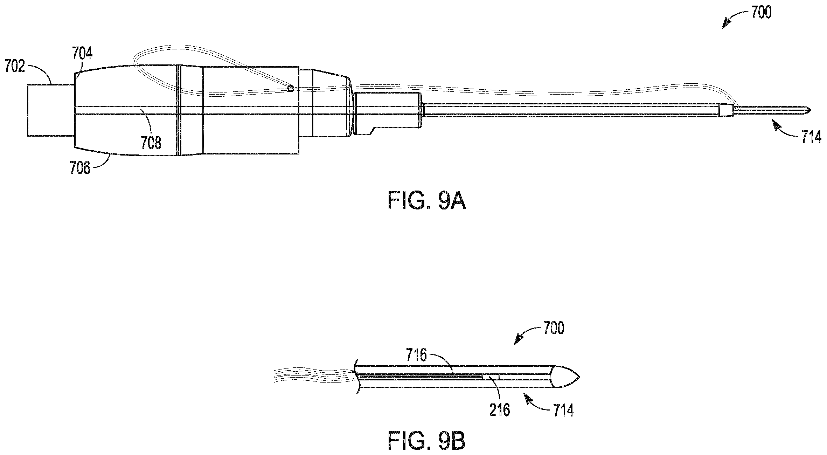

FIGS. 9A and 9B show another example of a repair device 700. The repair device 700 can be configured in a manner similar to those previously described but can differ in several respects. Rather than utilizing the push button as was the case with the repair device 100, the repair device 700 can include a thumb pusher 702 located at a proximal end 704 of a handle 706. The thumb pusher 702 can be coupled to the inserter 708 and can be configured to actuate the inserter 708 such as in the manner previously described. Sutures 710 of the repair device 700 can extend out a side surface of the handle 706.

As shown in FIG. 9B, a needle 714 of the repair device 700 can include a slit 716 or another aperture configured for the passage of at least one of the sutures 710. The slit 716 can have an elongated length along a longitudinal length of the needle 714 and can extend to an opening of the lumen of the needle 714. The slit 716 can be sized so as not to interfere with deployment of the anchors in one of the manners previously described. Thus, the slit 716 can be sized to be sufficiently small so the anchors cannot deform to pass therethrough.

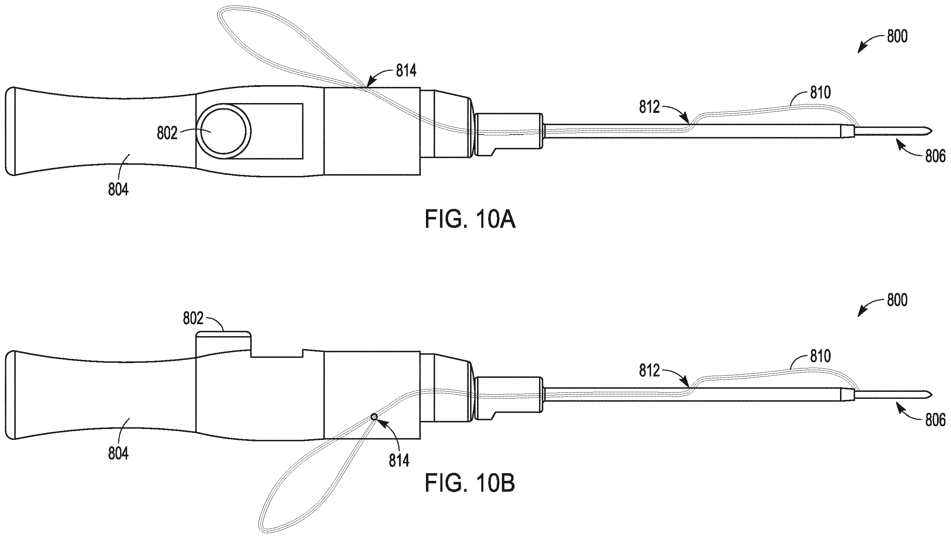

FIGS. 10A and 10B show yet another example of a repair device 800. The repair device 800 can be constructed in the manner of the repair device 700 but can include a push button 802 on a handle 804 in a manner as previously describe. The push button 802 can comprise part of the anchor actuation mechanism. The repair device 800 can include a needle 806 having a slit 808 similar to the slit 716. However, with the repair device 800, sutures 810 can pass through the slit 808 and can re-enter the needle 806 at an aperture 812 proximal of the slit 808. The sutures 810 can then pass through the handle 804 and can exit the handle 804 through a proximal end or through an aperture 814 in the side of the handle 804. The configuration of the repair device 800 with the slit 808, the aperture 812 and/or the aperture 814 can facilitate tensioning of the sutures 810 via torsional engagement of the sutures 810 during deployment of the first anchor and/or second anchor such as in one of the manners previously described.

FIGS. 10A and 10B, show another deployment scheme using the push button 802 with multiple positions. If the push button 802 has been advanced distally, the inserter can be advanced distally to a first position, In the first position, the first anchor can be forced distally out of the lumen of the needle 806 and can be been deployed at a suitable location in tissue. In the first position, the inserter can push the second anchor distally within the bore of the needle 806 such as to a position previously occupied by the first anchor. In some examples, the push button 802 can then be further advanced distally to a second position to advance the inserter further distally to force the second anchor from the needle 806 and into tissue.

In some examples, the repair device 800 can emit an audible click when the push button 802 depressed, advanced distally, etc. The click is produced by at least one outward-biased tab on the inserter snapping into a corresponding inward-facing step on the handle 804 or engaging with another component or feature. By snapping into the step, the outward-biased tab can block the inserter from moving proximally and/or beyond the position at which the snapping occurs, with respect to the handle 804 unless further actuated by the surgeon.

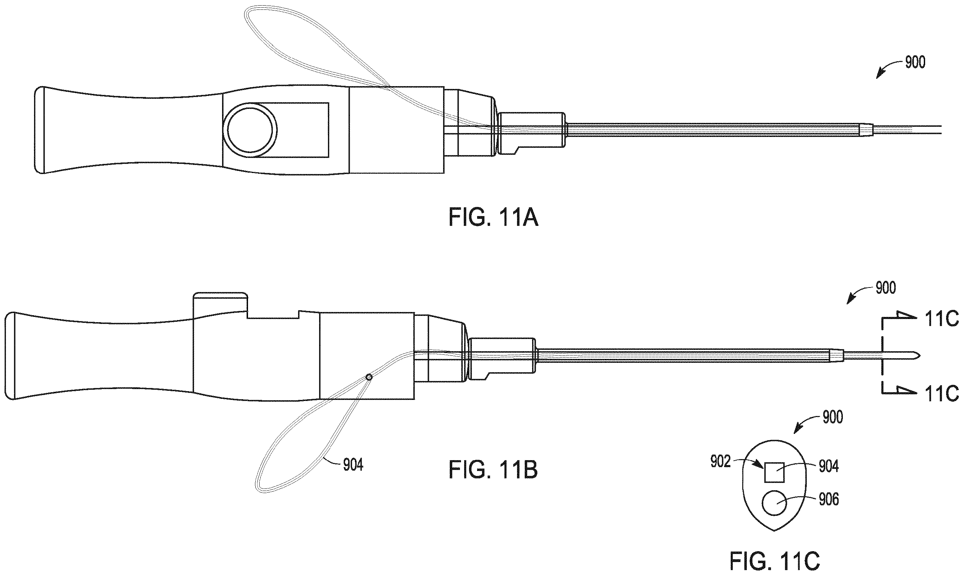

FIGS. 11A-11C shows a repair device 900 that be configured in a manner similar to that of FIGS. 9A-10B. However, the example of FIGS. 11A-11C can include a lumen 902 (FIG. 11C) configured for receiving sutures 904 therein. The lumen 902 can be separate from a second lumen 906 configured for receiving the anchors, inserter, etc.

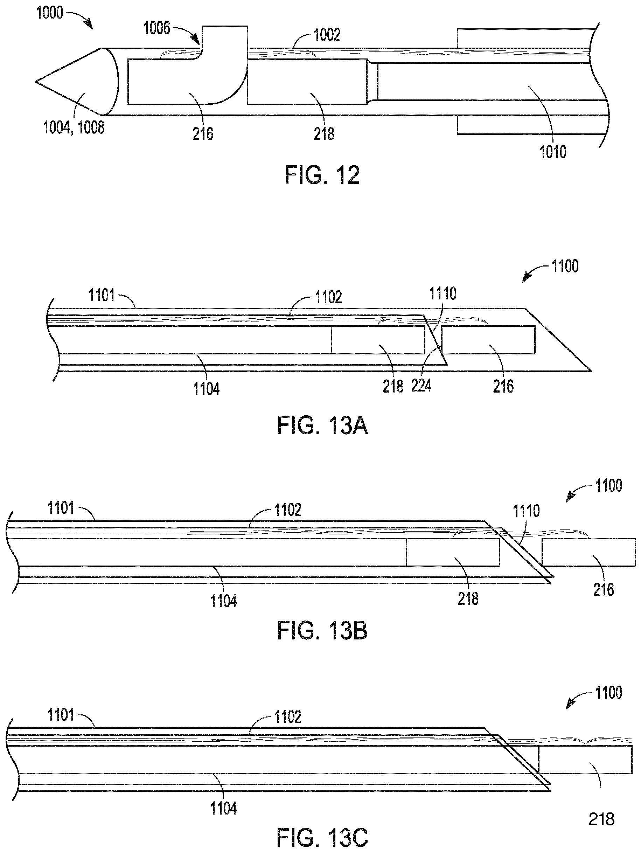

FIG. 12 shows a schematic of a distal end of a repair device 1000 according to another example. The repair device 1000 can include a needle 1002 having a lumen 1004 as previously described. The needle 1002 differs in that the needle 1002 can include a passage 1006 through a lateral side of the needle 1002. This passage 1006 can be proximal of and generally transverse to a distal opening 1008 of the lumen 1004. The passage 1006 can be configured to receive at least a portion of the first anchor 216 therein. The needle 1002 forming the passage 1006 can be tapered, chamfered or otherwise configured to selectively retain the first anchor 216 in the projecting state. The first anchor 216 can be deformed due to the soft material it is formed from to project laterally from the passage 1006 and side of the needle 1002 as illustrated in FIG. 12.

Once the needle 1002 is inserted into tissue and has begun to be removed therefrom, friction from contact between the first anchor 216 and the tissue can deploy the first anchor 216 from the passage 1006 into the tissue. Thus, the first anchor 216 can be deployed without use of a dedicated actuation mechanism such as an inserter 1010. Rather, deployment of the first anchor 216 can be entirely from friction with the tissue of the patient and the fist anchor 216. The second anchor 218 can be deployed into the tissue using the passage 1006 or by using mechanisms such as those described previously, (e.g., the inserter 1010 pushing the second anchor 218 from the needle 1002 from the distal opening 1008 of the lumen 1004).

FIGS. 13A-13C show a distal portion of another example of a repair device 1100. FIG. 13A shows the first anchor 216 and the second anchor 218 and the remainder of elements in the distal portion of the repair device 1100 prior to an initial deployment. The repair device 1100 differs from those of prior examples in that the repair device includes a tube pusher 1102 in addition to an inserter 1104 within the needle 1101.

The tube pusher 1102 can be positioned over the inserter 1104 within a lumen 1108 of the needle 1101. The tube pusher 1102 can have a distal end 1110 that extends distally beyond a distal end of the second anchor 218. The distal end 1110 can be tapered, angled, flared or otherwise configured to engage with the proximal end 224 of the first anchor 216. The distal end 1110 and other portions of the tube pusher 1102 can be configured to space the first anchor 216 from the second anchor 218. Additionally, the tube pusher 1102 can be sized with an inner cavity configured to receive the second anchor 218 therein.

FIG. 13B shows deployment of the first anchor 216. As such, the distal end 1110 of the tube pusher 1102 can be configured so that the tube pusher 1102 can push the first anchor 216 distally as the tube pusher 1102 advances distally within the lumen 1108 of the needle 1101. The tube pusher 1102 can be actuated from a proximal portion of the tube pusher 1102 such as by a push button or other feature of the handle (not shown). As shown in FIG. 13B, the inserter 1104 can be advanced with the advance of the tube pusher 1102. The inserter 1104 can engage and move the second anchor 218 in the manner previously described to advance the second anchor 218.

FIG. 13C shows deployment of the second anchor 218 from the tube pusher 1102 and needle 1101 via distal movement of the inserter 1104.

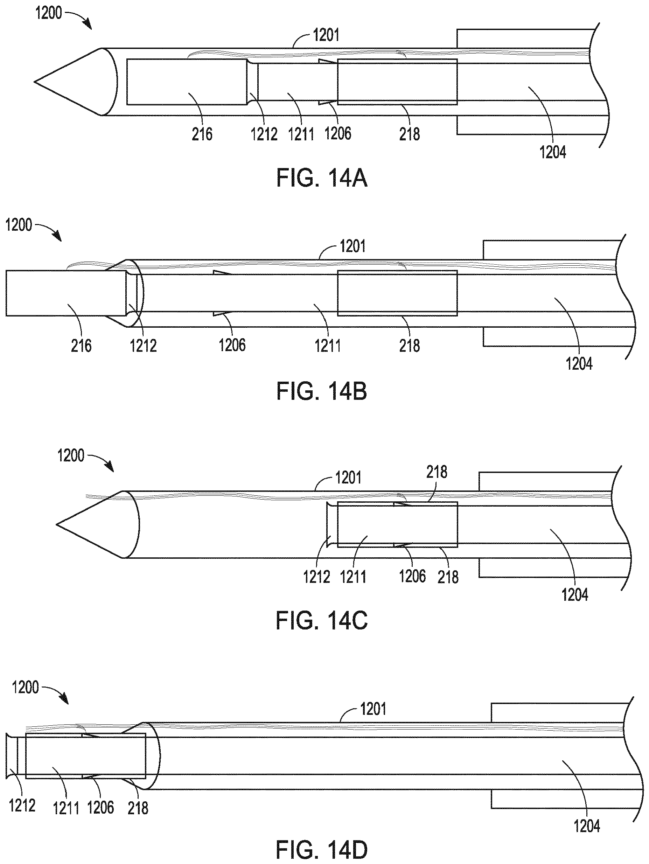

FIGS. 14A-14D show a distal portion of another example of a repair device 1200. FIG. 14A shows the repair device 1200 prior to deployment of the first anchor 216 and the second anchor 218 from a needle 1201 thereof. The repair device 1200 can be configured in a manner similar to those of the previously discussed. The repair device 1200 differs from prior devices in that an inserter 1204 thereof includes projections 1206. These projections 1206 can comprise teeth that extend laterally from a longitudinal extent of the inserter 1204. The projections 1206 can be configured (e.g., angled with sharp tips) to engage the second anchor 218 as further illustrated in FIG. 14D. The projections 1206 can be proximally spaced from a distal end 1212 of the inserter 1204 by an intermediate portion 1211. The intermediate portion 1211 can be configured to space the first anchor 216 from the second anchor 218. The distal end 1212 can be configured in the manner previously discussed (e.g. with a slightly larger diameter or different shape) to engage the proximal end 224 of the first anchor 216.

FIG. 14B shows deployment of the first anchor 216 from the needle 1201 with the distal end 1212 of the inserter 1204 engaging the first anchor 216 and the inserter 1204. Distal extension of the inserter 1204 facilitates this deployment.

FIG. 14C shows the inserter 1204 retracted proximally back into the needle 1201 to a position further proximal of the position of either FIG. 14A or FIG. 14B. In the retracted position of FIG. 14C, the projections 1206 can be received in an inner cavity of the second anchor 218. The projections 1206 can be angled so as not to engage with the inner cavity of the second anchor 218 while being retracted back toward the position of FIGS. 14C from the position of FIG. 14B. In some examples, the projections 1206 can be sized and shaped to slide along the inner cavity of the second anchor non-damagingly when the inserter 1204 is retracted proximally with respect to the second anchor.

FIG. 14D shows the inserter 1204 again advanced distally to deploy the second anchor 218. Initial distal advance of the inserter 1204 from the position of FIG. 14C causes the projections 1206, which are angled and provided with sharp tips, to engage/grasp the inner cavity of the second anchor 218. This engagement between the projections 1206 and the second anchor 218 at the inner cavity causes the second anchor 218 to be advanced distally until deployment of the second anchor 218 is achieved at the position of FIG. 14D.

## Claims

1. A tissue repair device (1100), comprising:
a needle (1101) defining a lumen (1108) that extends through a distal end of the needle adjacent a tip thereof;
a deformable first anchor (216) disposable in the lumen (1108) proximal to a distal end of the needle (1101);
a deformable second anchor (218) disposable in the lumen (1108) adjacent but proximal of the first anchor (216);
a member (1104) positioned within the lumen (1108) and engageable with at least one of the first anchor (216) or the second anchor (218), **characterized by**
a tube (1102) positioned over the member (1104) within the lumen (1108) of the needle (1101) and configured to engage the first anchor (216),
wherein the tube (1102) is moveable relative to the needle (1101) and the member (1104) to deploy the first anchor (216) from the lumen (1108) by pushing the first anchor (216) out of the repair device (1100), and wherein, after
deployment of the first anchor (216), the member (1104) is moveable relative to the tube (1102) and the needle (1101) to deploy the second anchor (218) through at least a portion of an inner cavity of the tube (1102) and from the lumen (1108) of the needle (1101).

2. The repair device of claim 1, wherein the tube receives the second anchor (218) within an inner cavity thereof and has a distal end that engages the first anchor (216).

3. The repair device of claim 1, wherein the needle is selectively retractable relative to the tube and the member to deploy the at least one of the first anchor (216) or the second anchor (218) from the lumen.

4. The repair device of claim 1, wherein the needle is selectively retractable relative to the member to deploy the at least one of the first anchor (216) or the second anchor (218) from the lumen.

5. The repair device of claim 1, wherein the needle has a passageway proximal of the distal end thereof, wherein the first anchor (216) is disposable in the lumen to protrude from the passageway and the needle so as to be engageable with a tissue of a patient when the needle is inserted into the tissue, and wherein the first anchor (216) is configured to deploy into the tissue through the passageway.

6. The repair device of any one of claims 1-5, wherein the first anchor (216) and the second anchor (218) are coupled together with a suture (208) and wherein one of: the suture (208) is positioned and extends through a second lumen of the needle, the suture (208) extends through the lumen and out a proximal end of the repair device, or the suture (208) passes through a slit extending along a longitudinal length of the needle proximal of the distal end.

7. The repair device(100) of any one of claims 1-6, wherein the member is configured to pass around the second anchor (218) to engage the first anchor (216).

8. The repair device of any one of claims 1-7, further comprising:
a handler fixedly coupled to a proximal end of the needle; and
a push button disposed on an exterior of the handle and coupled to the member,
the push button being moveable proximally and distally with respect to the handle to move one of the member, wherein the push button has a first position to deploy the first anchor (216) from the lumen and is depressible to further move to a second position to deploy the second anchor (218) from the lumen.

9. The repair device of any one of claims 1-8, further comprising a lumen device (110) configured to insert in a tissue of a patient, wherein the lumen device (110) is configured to receive the needle for passage of the needle through the tissue.

10. The repair device of any one of claims 1-9, wherein a distal end portion to the tip (201) of the needle (102) is angled relative to a proximal portion of the needle (102).

11. The tissue repair device of any one of claims 1-10, wherein the needle is selectively retractable relative to the member to deploy sequentially the first anchor and then the second anchor from the lumen.

## Patentansprüche

1. Gewebereparaturvorrichtung (1100), umfassend:
eine Nadel (1101), die ein Lumen (1108) definiert, das sich durch ein distales Ende der Nadel benachbart zu einer Spitze davon erstreckt;
einen verformbaren ersten Anker (216), der in dem Lumen (1108) proximal zu einem distalen Ende der Nadel (1101) anordenbar ist,
einen verformbaren zweiten Anker (218), der in dem Lumen (1108) benachbart zu dem ersten Anker (216), aber proximal davon anordenbar ist,
ein Element (1104), das innerhalb des Lumens (1108) positioniert ist und mit mindestens einem von dem ersten Anker (216) oder dem zweiten Anker (218) in Eingriff bringbar ist,
**gekennzeichnet durch**
ein Rohr (1102), das über dem Element (1104) innerhalb des Lumens (1108) der Nadel (1101) positioniert ist und dazu konfiguriert ist, den ersten Anker (216) in Eingriff zu nehmen,
wobei das Rohr (1102) relativ zu der Nadel (1101) und dem Element (1104) bewegbar ist, um den ersten Anker (216) aus dem Lumen (1108) durch Schieben des ersten Ankers (216) aus der Reparaturvorrichtung (1100) heraus auszufahren, und wobei, nach dem Ausfahren des ersten Ankers (216), das Element (1104) relativ zu dem Rohr (1102) und der Nadel (1101) bewegbar ist, um den zweiten Anker (218) durch mindestens einen Abschnitt eines inneren Hohlraums des Rohrs (1102) und aus dem Lumen (1108) der Nadel (1101) auszufahren.

2. Reparaturvorrichtung nach Anspruch 1, wobei das Rohr den zweiten Anker (218) innerhalb eines inneren Hohlraums davon aufnimmt und ein distales Ende aufweist, das den ersten Anker (216) in Eingriff nimmt.

3. Reparaturvorrichtung nach Anspruch 1, wobei die Nadel selektiv relativ zu dem Rohr und dem Element zurückziehbar ist, um den mindestens einen von dem ersten Anker (216) oder dem zweiten Anker (218) aus dem Lumen auszufahren.

4. Reparaturvorrichtung nach Anspruch 1, wobei die Nadel selektiv relativ zu dem Element zurückziehbar ist, um den mindestens einen von dem ersten Anker (216) oder dem zweiten Anker (218) aus dem Lumen auszufahren.

5. Reparaturvorrichtung nach Anspruch 1, wobei die Nadel einen Durchgang proximal des distalen Endes davon aufweist, wobei der erste Anker (216) so in dem Lumen anordenbar ist, dass er aus dem Durchgang und der Nadel hervorsteht, sodass er mit einem Gewebe eines Patienten in Eingriff bringbar ist, wenn die Nadel in das Gewebe eingesetzt wird, und wobei der erste Anker (216) dazu konfiguriert ist, durch den Durchgang in das Gewebe ausgefahren zu werden.

6. Reparaturvorrichtung nach einem der Ansprüche 1-5, wobei der erste Anker (216) und der zweite Anker (218) mit einem Nahtmaterial (208) miteinander gekoppelt sind und
wobei eines von Folgendem gilt: das Nahtmaterial (208) ist durch ein zweites Lumen der Nadel positioniert und erstreckt sich dadurch, das Nahtmaterial (208) erstreckt sich durch das Lumen und aus einem proximalen Ende der Reparaturvorrichtung heraus oder das Nahtmaterial (208) verläuft durch einen Schlitz, der sich entlang einer längsgerichteten Länge der Nadel proximal des distalen Endes erstreckt.

7. Reparaturvorrichtung(100) nach einem der Ansprüche 1-6, wobei das Element dazu konfiguriert ist, um den zweiten Anker (218) herum zu verlaufen, um den ersten Anker (216) in Eingriff zu nehmen.

8. Reparaturvorrichtung nach einem der Ansprüche 1-7, ferner umfassend:
einen Griff, der fest mit einem proximalen Ende der Nadel gekoppelt ist, und
einen Druckknopf der an einer Außenseite des Griffs angeordnet und mit dem Element gekoppelt ist,
wobei der Druckknopf proximal und distal in Bezug auf den Griff bewegbar ist, um eines von dem Element zu bewegen, wobei der Druckknopf eine erste Position aufweist, um den ersten Anker (216) aus dem Lumen auszufahren, und niederdrückbar ist, um sich ferner in eine zweite Position zu bewegen, um den zweiten Anker (218) aus dem Lumen auszufahren.

9. Reparaturvorrichtung nach einem der Ansprüche 1-8, ferner eine Lumenvorrichtung (110) umfassend, die zum Einsetzen in ein Gewebe eines Patienten konfiguriert ist, wobei die Lumenvorrichtung (110) zum Aufnehmen der Nadel zur Durchführung der Nadel durch das Gewebe konfiguriert ist.

10. Reparaturvorrichtung nach einem der Ansprüche 1-9, wobei ein distaler Endabschnitt zu der Spitze (201) der Nadel (102) relativ zu einem proximalen Abschnitt der Nadel (102) abgewinkelt ist.

11. Gewebereparaturvorrichtung nach einem der Ansprüche 1-10, wobei die Nadel selektiv relativ zum Element zurückziehbar ist, um nacheinander den ersten Anker und dann den zweiten Anker aus dem Lumen auszufahren.

## Revendications

1. Dispositif de réparation de tissus (1100), comprenant :
une aiguille (1101) définissant une lumière (1108) qui s'étend à travers l'extrémité distale de l'aiguille adjacente à la pointe de celle-ci ;
un premier ancrage déformable (216) pouvant être disposé dans la lumière (1108) à proximité de l'extrémité distale de l'aiguille (1101) ;
un second ancrage déformable (218) pouvant être disposé dans la lumière (1108) adjacent mais à proximité du premier ancrage (216) ;
un élément (1104) positionné à l'intérieur de la lumière (1108) et pouvant se mettre en prise avec au moins un ancrage parmi le premier ancrage (216) ou le second ancrage (218),
**caractérisé par** un tube (1102) positionné sur l'élément (1104) à l'intérieur de la lumière (1108) de l'aiguille (1101) et conçu pour se mettre en prise avec le premier ancrage (216), ledit tube (1102) étant mobile par rapport à l'aiguille (1101) et à l'élément (1104) de manière à déployer le premier ancrage (216) à partir de la lumière (1108) en poussant le premier ancrage (216) hors du dispositif de réparation (1100), et après déploiement du premier ancrage (216), ledit élément (1104) étant mobile par rapport au tube (1102) et à l'aiguille (1101) de manière à déployer le second ancrage (218) à travers au moins une partie d'une cavité interne du tube (1102) et de la lumière (1108) de l'aiguille (1101).

2. Dispositif de réparation selon la revendication 1, ledit tube recevant le second ancrage (218) à l'intérieur d'une cavité interne de celui-ci et comportant une extrémité distale qui se met en prise avec le premier ancrage (216).

3. Dispositif de réparation selon la revendication 1, ladite aiguille étant rétractable de façon sélective par rapport au tube et à l'élément de manière à déployer ledit au moins un ancrage parmi le premier ancrage (216) ou le second ancrage (218) à partir de la lumière.

4. Dispositif de réparation selon la revendication 1, ladite aiguille étant rétractable de façon sélective par rapport à l'élément de manière à déployer au moins un ancrage parmi le premier ancrage (216) ou le second ancrage (218) à partir de la lumière.

5. Dispositif de réparation selon la revendication 1, ladite aiguille comportant un passage proximal de son extrémité distale, ledit premier ancrage (216) pouvant être disposé dans la lumière de manière à faire saillie depuis le passage et l'aiguille de manière à pouvoir se mettre en prise avec un tissu d'un patient lorsque l'aiguille est insérée dans le tissu, et ledit premier ancrage (216) étant conçu pour se déployer dans le tissu par l'intermédiaire du passage.

6. Dispositif de réparation selon l'une quelconque des revendications 1 à 5, ledit premier ancrage (216) et ledit second ancrage (218) étant couplés ensemble avec une suture (208) et l'un des éléments suivants : la suture (208) étant positionnée et s'étendant à travers une seconde lumière de l'aiguille, la suture (208) s'étendant à travers la lumière et sortant de l'extrémité proximale du dispositif de réparation, ou la suture (208) passant à travers une fente s'étendant le long de la longueur longitudinale de l'aiguille à proximité de l'extrémité distale.

7. Dispositif de réparation (100) selon l'une quelconque des revendications 1 à 6, ledit élément étant conçu pour passer autour du second ancrage (218) de manière à venir en prise avec le premier ancrage (216).

8. Dispositif de réparation selon l'une quelconque des revendications 1 à 7, comprenant en outre :
une poignée couplée de manière fixe à une extrémité proximale de l'aiguille ; et
un bouton-poussoir disposé sur l'extérieur de la poignée et couplé à l'élément, le bouton-poussoir étant mobile de manière proximale et de manière distale par rapport à la poignée de manière à déplacer l'un des éléments, ledit bouton-poussoir comportant une première position destinée à déployer le premier ancrage (216) à partir de la lumière et pouvant être enfoncé pour se déplacer en outre vers une seconde position destinée à déployer le second ancrage (218) à partir de la lumière.

9. Dispositif de réparation selon l'une quelconque des revendications 1 à 8, comprenant en outre un dispositif à lumière (110) conçu pour être inséré dans un tissu d'un patient, ledit dispositif à lumière (110) étant conçu pour recevoir l'aiguille en vue du passage de l'aiguille à travers le tissu.

10. Dispositif de réparation selon l'une quelconque des revendications 1 à 9, l'extrémité distale de la partie de la pointe (201) de l'aiguille (102) étant inclinée par rapport à une partie proximale de l'aiguille (102).

11. Dispositif de réparation de tissus selon l'une quelconque des revendications 1 à 10, ladite aiguille étant rétractable de façon sélective par rapport à l'élément de manière à déployer de façon séquentielle le premier ancrage puis le second ancrage à partir de la lumière.
